(19) Europäisches Patentamt / European Patent Office / Office européen des brevets

(11) **EP 4 260 741 A1**

(12) **EUROPEAN PATENT APPLICATION**
published in accordance with Art. 153(4) EPC

(43) Date of publication:
**18.10.2023 Bulletin 2023/42**

(21) Application number: **20965836.8**

(22) Date of filing: **14.12.2020**

(51) International Patent Classification (IPC):
*A24F 40/65* (2020.01)     *A61B 5/00* (2006.01)
*A61B 5/16* (2006.01)

(52) Cooperative Patent Classification (CPC):
**A24F 40/65; A61B 5/00; A61B 5/16**

(86) International application number:
**PCT/JP2020/046519**

(87) International publication number:
**WO 2022/130448 (23.06.2022 Gazette 2022/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Japan Tobacco Inc.**
**Tokyo 105-6927 (JP)**

(72) Inventors:
• **KATO, Masato**
  **Tokyo 130-8603 (JP)**
• **SHIMADA, Yurina**
  **Tokyo 130-8603 (JP)**

(74) Representative: **Hoffmann Eitle**
**Patent- und Rechtsanwälte PartmbB**
**Arabellastraße 30**
**81925 München (DE)**

(54) **INFORMATION PROCESSING DEVICE, INFORMATION PROCESSING METHOD, AND INFORMATION PROCESSING SYSTEM**

(57) The present invention provides an information processing device, an information processing method, and an information processing system with which the user can understand a change in their state of health associated with the use of an inhalation device. The information processing device according to the present disclosure is provided with: a first acquirer that acquires usage information about an inhalation device by the user from the inhalation device; a second acquirer that acquires health information about the user from a wearable terminal; a processor that associates the health information with the usage information; and an output unit that generates and outputs a daily activity screen in response to a request from the user, the activity screen including a display of a graph in which a record of the use of the inhalation device based on the usage information and variations in a user state based on the health information are illustrated over time.

Fig. 1

## Description

TECHNICAL FIELD

The present disclosure relates to an information processing device, an information processing method, and an information processing system.

BACKGROUND ART

Inhalation products with which a user inhales flavors are widely known. Such inhalation products include aerosol-generating devices. An aerosol-generating device refers to an electronic device for inhaling a generated aerosol, including but not limited to electronic tobacco products, heated tobacco products, and medical nebulizers, for example.

Recently, an aerosol-generating device equipped with a biometric sensor is also known. Such an aerosol-generating device can acquire biometric data about a user by acquiring a bioelectric signal from the user, for example. Also, among such aerosol-generating devices, there are devices that use biometric data acquired by the biometric sensor to determine an indicator (for example, a stress indicator) indicating the user's state of health.

CITATION LIST

PATENT LITERATURE

Patent Literature 1: International Publication No. WO 2019/175810
Patent Literature 2: International Publication No. WO 2016/199065
Patent Literature 3: International Publication No. WO 2018/098371

SUMMARY OF INVENTION

TECHNICAL PROBLEM

An objective of the present disclosure is to provide a system that associate health information about a user with usage information about the use of an inhalation device by the user, thereby enabling the user to understand a change in their state of health associated with the use of the inhalation device.

SOLUTION TO PROBLEM

To address the above problem, in a first aspect, an information processing device is provided. The information processing device is provided with: a first acquirer that acquires usage information about the use of an inhalation device by a user from the inhalation device; a second acquirer that acquires health information about the user from a wearable terminal; a processor that associates the health information with the usage information; and an output unit that generates and outputs a daily activity screen in response to a request from the user, the activity screen including a display of a graph in which a record of the use of the inhalation device based on the usage information and variations in a user state based on the health information are illustrated over time.

According to an information processing device of a second aspect, in the information processing device of the first aspect, the usage information includes a usage period of the inhalation device, and the processor is configured to associate the health information specified at a time later than the usage period with the record of the use of the inhalation device.

According to an information processing device of a third aspect, in the information processing device of the first or second aspect, the processor is configured to associate with the usage information a stress value calculated on a basis of a heart rate of the user monitored by the wearable terminal, and the variations in the user state are variations in the stress value.

According to an information processing device of a fourth aspect, in the information processing device of the third aspect, the processor is further configured to determine a change between the stress value when the inhalation device is used and the stress value after the inhalation device is used with respect to the record of the use of the inhalation device, and the output unit is further configured to output the change in the stress value with respect to the record of the use of the inhalation device.

According to an information processing device of a fifth aspect, in the information processing device of the fourth aspect, the usage information includes a usage end time of the inhalation device, and the stress value after the inhalation device is used is the stress value after the passage of a predetermined first time period from the usage end time.

According to an information processing device of a sixth aspect, in the information processing device of the fifth aspect, the usage information further includes a usage start time of the inhalation device, and the stress value when the inhalation device is used is the stress value at the usage start time or the usage end time.

According to an information processing device of a seventh aspect, in the information processing device of the fifth aspect, the usage information further includes a usage start time of the inhalation device, and the stress value when the inhalation device is used is a maximum value or a minimum value of the stress value from the usage start time until the passage of a predetermined second time period.

According to an information processing device of an eighth aspect, in the information processing device of any of the first to seventh aspects, the inhalation device is an aerosol-generating device, the usage information includes a puff count for the aerosol-generating device, and the processor is configured to associate the health information with the puff count for a series of puff operations by the user.

According to an information processing device of a ninth aspect, in the information processing device of the eighth aspect, the usage information includes a puff operation time from a beginning to an end of the series of puff operations by the user, and the output unit is further configured to output a daily total time of the puff operation time.

According to an information processing device of a 10th aspect, in the information processing device of the eighth or ninth aspect, the output unit is further configured to output a consumption quantity of the aerosol-generating device calculated on the basis of a daily total of the puff count.

In an 11th aspect, an information processing method is provided. The information processing method includes: acquiring usage information about a use of an inhalation device by a user from the inhalation device and acquiring health information about the user from a wearable terminal; associating the health information with the usage information; and generating and outputting a daily activity screen in response to a request from the user, the activity screen including a display of a graph in which a record of the use of the inhalation device based on the usage information and variations in a user state based on the health information are illustrated over time.

According to an information processing method of a 12th aspect, in the information processing method of the 11th aspect, the usage information includes a usage period of the inhalation device, and the associating step is configured to associate the health information specified at a time later than the usage period with the record of the use of the inhalation device.

According to an information processing method of a 13th aspect, in the information processing method of the 11th or 12th aspect, the associating step is configured to associate with the usage information a stress value calculated on a basis of a heart rate of the user monitored by the wearable terminal, and the variations in the user state are variations in the stress value.

According to an information processing method of a 14th aspect, in the information processing method of the 13th aspect, the associating step further includes determining a change between the stress value when the inhalation device is used and the stress value after the inhalation device is used with respect to the record of the use of the inhalation device, and the outputting step further includes outputting the change in the stress value with respect to the record of the use of the inhalation device.

According to an information processing method of a 15th aspect, in the information processing method of the 14th aspect, the usage information includes a usage end time of the inhalation device, and the stress value after the inhalation device is used is the stress value after the passage of a predetermined first time period from the usage end time.

According to an information processing method of a 16th aspect, in the information processing method of the 15th aspect, the usage information further includes a usage start time of the inhalation device, and the stress value when the inhalation device is used is the stress value at the usage start time or the usage end time.

According to an information processing method of a 17th aspect, in the information processing method of any of the 11th to 16th aspects, the inhalation device is an aerosol-generating device, the usage information includes a puff count for the aerosol-generating device, the associating step is configured to associate the health information with the puff count for a series of puff operations by the user, and the outputting step further includes outputting at least one of a daily total of the puff count or a consumption quantity of the aerosol-generating device calculated on a basis of the daily total of the puff count.

According to an information processing method of an 18th aspect, in the information processing method of the 17th aspect, the usage information includes a puff operation time from a beginning to an end of a series of puff operations by the user, and the outputting step further includes outputting a daily total time of the puff operation time.

In a 19th aspect, an information processing system is provided. The information processing system is provided with a user terminal and a server, the user terminal including: a first acquirer that acquires usage information about a use of an inhalation device by a user from the inhalation device; a second acquirer that acquires health information about the user from a wearable terminal; and an output unit that generates and outputs a daily activity screen in response to a request from the user, and the server including a processor that associates the health information with the usage information, the activity screen including a display of a graph in which a record of the use of the inhalation device based on the usage information and variations in a user state based on the health information are illustrated over time.

According to an information processing method of a 20th aspect, in the information processing system of the 19th aspect, the server is further provided with a ranking determiner that determines a ranking of the user on a basis of the health information associated with the usage information, and the activity screen to be displayed by the output unit of the user terminal is configured to display the ranking of the user.

BRIEF DESCRIPTION OF DRAWINGS

[Fig. 1] Fig. 1 is a schematic configuration diagram of an overall system.
[Fig. 2] Fig. 2 is a schematic diagram of a configuration example of an aerosol-generating device.

[Fig. 3] Fig. 3 is a schematic diagram of a hardware configuration example of a user terminal.

[Fig. 4] Fig. 4 is a schematic diagram of a hardware configuration example of a server.

[Fig. 5] Fig. 5 is a schematic diagram of a hardware configuration example of a wearable terminal.

[Fig. 6] Fig. 6 is a schematic functional configuration diagram of an information processing system according to a first embodiment.

[Fig. 7] Fig. 7 is a schematic process flowchart for the information processing system according to the first embodiment.

[Fig. 8] Fig. 8 is one example of a stress curve indicating variations in a user's stress.

[Fig. 9] Fig. 9 is an example of an activity screen image displayed on a user terminal.

[Fig. 10A] Fig. 10A is an example of a graph displayed on the activity screen.

[Fig. 10B] Fig. 10B is an example of a graph displayed on the activity screen.

[Fig. 10C] Fig. 10C is an example of a graph displayed on the activity screen.

[Fig. 11] Fig. 11 is an example of an activity screen image displayed on a user terminal.

[Fig. 12] Fig. 12 is a schematic functional configuration diagram for an information processing system according to a second embodiment.

[Fig. 13] Fig. 13 is a schematic process flowchart for the information processing system according to the second embodiment.

[Fig. 14] Fig. 14 is an example of a ranking screen image displayed on a user terminal.

DESCRIPTION OF EMBODIMENTS

Hereinafter, an information processing device, an information processing method, and an information processing system according to embodiments of the present disclosure will be described with reference to the accompanying drawings. In the accompanying drawings, the same or similar elements are denoted with the same or similar reference signs, and duplicate descriptions of the same or similar elements may be omitted from the description of each embodiment. Also, the features indicated in each embodiment are also applicable to other embodiments insofar as the embodiments do not contradict each other. Furthermore, the drawings are schematic and do not necessarily correspond to actual dimensions, proportions, and the like. The drawings may also include portions where the dimensional relationships and ratios differ from each other.

According to the present embodiment, by associating health information about a user with usage information about the use of an inhalation device by the user, a change in the state of health associated with the use of the inhalation device can be presented to the user effectively. In particular, a reduction in the stress felt by the user before and after the user uses the inhalation device can be presented to the user in a visual way. In the following description, the inhalation device is assumed to be an aerosol-generating device, particularly a heated flavor inhaler, but is not limited thereto.

<1. Overall system>

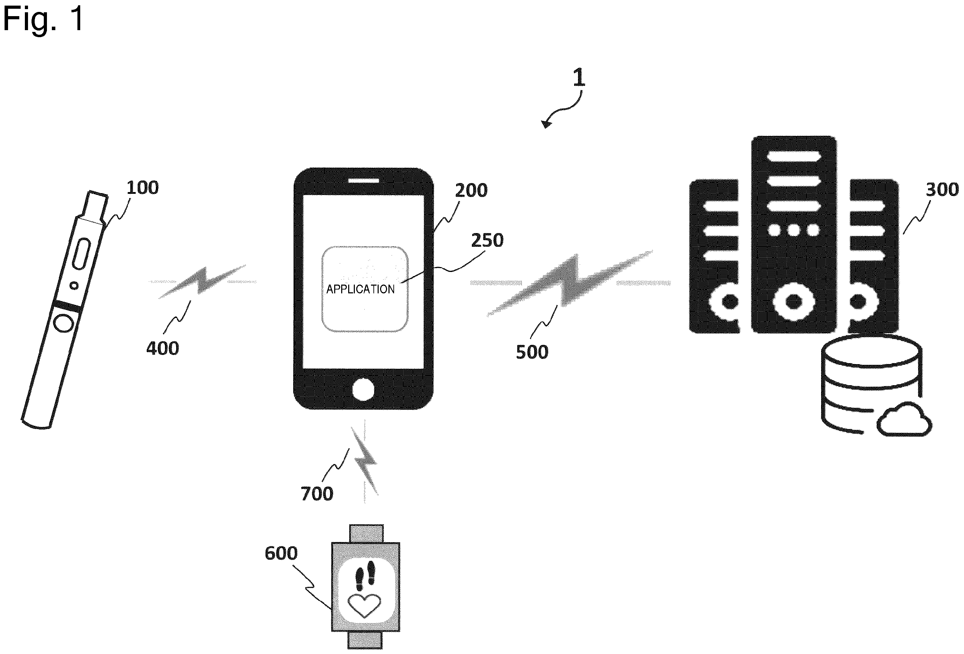

Fig. 1 is a schematic configuration diagram of an overall system 1 related to an embodiment of the present disclosure. The overall system 1 is provided with an aerosol-generating device 100, a user terminal 200, a server 300, a wearable terminal 600, and networks 400, 500, and 700.

The aerosol-generating device 100 is an electronic device that generates an aerosol or an aerosol with added flavor to be inhaled by a user. The user terminal 200 executes an application 250 for acquiring usage information about the use of the aerosol-generating device 100 by the user and health information about the user, and associating this information to thereby present to the user a change in the state of health associated with the use of the aerosol-generating device 100. The server 300 cooperates with the application 250 on the user terminal 200 to manage various data related to the user and the application 250 and the like. Note that the server 300 may also execute some of the processes by the application 250. The wearable terminal 600 is an information processing device which is worn on the body of the user and which acquires an activity level including biometric information.

The network 400 connects the aerosol-generating device 100 and the user terminal 200. The connection may be a wireless connection or a wired connection. The network 500 connects the user terminal 200 and the server 300 wirelessly, and may be the Internet in one example. The network 700 connects the wearable terminal 600 and the user terminal 200. Like the network 400, the connection may be a wireless connection or a wired connection.

Hereinafter, the configuration of each of the aerosol-generating device 100, the user terminal 200, the server 300, and the wearable terminal 600 provided in the system 1 will be described.

<1-1. Aerosol-generating device>

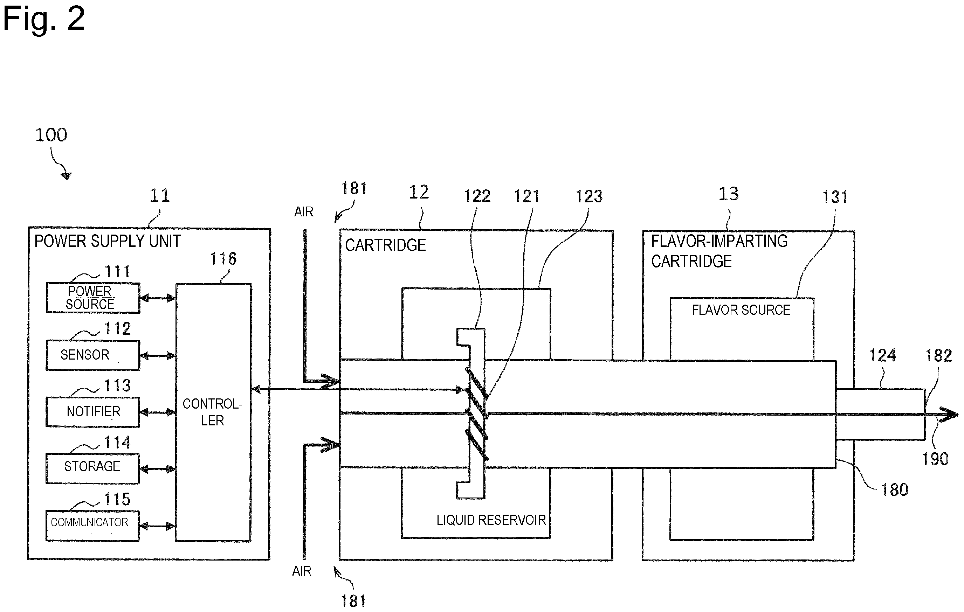

Fig. 2 is a schematic diagram of a configuration example of the aerosol-generating device 100. The aerosol-generating device 100 may be a heated flavor inhaler, for example, and may include any of various inhalation devices that generate an aerosol or an aerosol with added flavor to be inhaled by the user. Moreover, the generated inhalation component may also contain a gas such as invisible vapor in addition to an aerosol.

The aerosol-generating device 100 includes a power supply unit 11, a cartridge 12, and a flavor-imparting cartridge 13. Functionally, the power supply unit 11 includes a power source 111, a sensor 112, a notifier 113, storage 114, a communicator 115, and a controller 116. The cartridge 12 includes a heater 121, a liquid channel 122, and a liquid reservoir 123. The flavor-imparting cartridge 13 includes a flavor source 131 and a mouthpiece 124. An air channel 180 is formed in the cartridge 12 and the flavor-imparting cartridge 13.

The power source 111 stores electric power. The power source 111 supplies electric power to each component of the aerosol-generating device 100 on the basis of control by the controller 116. The power source 111 is formed from a rechargeable battery such as a lithium-ion secondary cell, for example.

The sensor 112 acquires various information related to operations by the aerosol-generating device 100. As an example, the sensor 112 may include a button, switch, or the like, and detects when the aerosol-generating device 100 is powered on or off. The sensor 112 may also include a pressure sensor such as a microphone condenser, a flow rate sensor, a temperature sensor, or the like, and acquire various values (such as temperature information, battery information, and malfunction information, for example) related to inhalation operations (puff operations) by the user.

Furthermore, the sensor 112 is achieved with an inhalation sensor and acquires the number of puff operations (puff count) by the user. The sensor 112 also cooperates with a timer (not illustrated) provided in the controller 116 to acquire a puff operation time over a series of puff operations. In addition, the sensor 112 may be achieved with a contact sensor or the like, and by detecting the attachment/detachment of the flavor-imparting cartridge 13, acquire a consumption quantity (such as the number of times a cartridge is used or replaced, for example) consumed through inhalation operations by the user. Alternatively, the consumption quantity of the flavor-imparting cartridge 13 may be calculated from puff information such as the puff count of the aerosol-generating device 100. For example, a series of 15 puffs by the user may be associated with the consumption of one flavor-imparting cartridge 13.

In this way, by acquiring the puff count, the puff operation time, and/or the consumption quantity of the flavor-imparting cartridge 13, a usage history of the aerosol-generating device 100 by the user can be specified. In the following, the consumption quantity of the aerosol-generating device 100 is assumed to be related to the puff count of the aerosol-generating device 100 and/or the used number of the flavor-imparting cartridge 13. However, the consumption quantity is not limited to the above and may also include the number of the cartridge 12 instead of, or in addition to, the used number of the flavor-imparting cartridge 13, for example.

Information related to puff operations by the user and information related to a usage history of the aerosol-generating device 100 may be stored in the storage 114 as usage information about the aerosol-generating device 100 together with corresponding times specified by the timer (not illustrated) provided in the controller 116.

The notifier 113 notifies the user about various information related to operations by the aerosol-generating device 100. For example, the notifier 113 is configured as a light-emitting device (for example, an LED) that emits light, a display device that displays images, a sound output device that outputs sound, a vibration device that vibrates, or the like.

The storage 114 stores various information related to operations by the aerosol-generating device 100. For example, the storage 114 includes a non-volatile storage medium such as flash memory. The storage 114 also stores programs such as firmware in addition to computer-executable instructions for causing the aerosol-generating device 100 to operate.

The communicator 115 is a communication interface capable of performing communication conforming to any given wired or wireless communication standard. For example, in the case of wireless communication, Wi-Fi(R), Bluetooth(R), or the like may be adopted. Also, in the case of wired communication, a data communication cable is connected through an external connection terminal such as Micro-USB, for example.

The communicator 115 inputs/outputs data related to operations by the aerosol-generating device 100 with respect to an external device such as the user terminal 200. Note that the communicator 115 may be provided internally in the aerosol-generating device 100, or may be provided in a communication device that can be removably attached to the aerosol-generating device 100 and function together with the aerosol-generating device.

The controller 116 functions as a computational processing device and control device, and controls overall operations inside the aerosol-generating device 100 by following various programs. The controller 116 is achieved by an electronic circuit such as a central processing unit (CPU) or a microprocessor, for example.

The heater 121 heats an aerosol source to atomize the aerosol source and generate an aerosol. For example, the heater 121 is configured as a coil that is wound around the liquid channel 122. The heater 121 generates heat when supplied with power from the power source 111. Additionally, when the heater 121 generates heat, the aerosol source held in the liquid channel 122 is heated and atomized, and an aerosol is generated.

The aerosol source, that is, the liquid stored in the liquid reservoir 123, is channeled from the liquid reservoir 123 and held in the liquid channel 122. For example, the liquid channel 122 may be a wick formed by twisting fiber materials such as glass fiber or other porous materials such as porous ceramics. In this case, the aerosol source stored in the liquid reservoir 123 is channeled by the capillary effect of the wick.

The liquid reservoir 123 stores an aerosol source. For example, the aerosol source may be a polyhydric alcohol such as glycerin or propylene glycol and a liquid such as water. The aerosol source may also contain tobacco-derived or non-tobacco-derived flavor components.

The air channel 180 is a channel for air to be inhaled by the user. The air channel 180 has a tubular structure with an air inflow hole 181 serving as an inlet for air into the air channel 180 and an air outflow hole 182 serving as an outlet for air from the air channel 180 on either end. Air flowing in from the air inflow hole 181 in association with inhalation by the user is mixed with the aerosol generated by the heater 121, and as indicated by the arrow 190, passes through the flavor source 131 and is transported to the air outflow hole 182. When the fluid mixture of aerosol and air passes through the flavor source 131, the flavor component contained in the flavor source 131 is imparted to the aerosol.

The mouthpiece 124 is a member that is put into the user's mouth during inhalation. The air outflow hole 182 is disposed in the mouthpiece 124. The user puts the mouthpiece 124 into the user's mouth and inhales, and can thereby draw in the fluid mixture of aerosol and air into the oral cavity.

The above describes a configuration example of the aerosol-generating device 100. It is understood by a person skilled in the art that the configuration of the aerosol-generating device 100 is not limited to the above.

<1-2. User terminal>

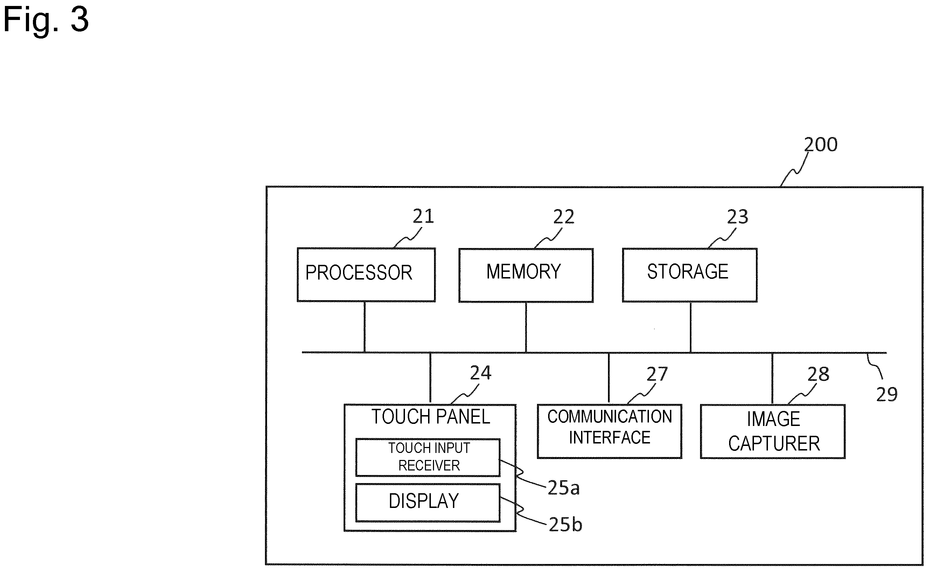

Fig. 3 is a schematic diagram of a hardware configuration example of the user terminal 200. For example, the user terminal 200 may be an electronic device such as a smartphone, a tablet terminal, a laptop computer, a personal computer, a feature phone, or a personal digital assistant (PDA). Note that a smartphone is assumed in the example of the user terminal 200 illustrated in Fig. 3.

The user terminal 200 is provided with a processor 21, a memory 22, storage 23, a touch panel 24, a communication interface 27, and an image capturer 28. These elements are electrically connected to each other via a bus 29.

The processor 21 is a computational processing device that controls information processing, controls the exchange of data between elements, and executes an information processing method according to an embodiment. For example, the processor 21 is a CPU that executes a program such as the application 250 stored in the storage 23 and loaded in the memory 22 to execute an information processing method according to an embodiment. Moreover, the processor 21 of the user terminal 200 may also be provided with a graphics processing unit (GPU) or the like in addition to a CPU.

The memory 22 includes main memory achieved with a volatile storage device such as dynamic random access memory (DRAM) and auxiliary memory achieved with a non-volatile storage device such as flash memory or a hard disk drive (HDD). The memory 22 is used as a work area for the processor 21 and the like, and stores a basic input/output system (BIOS) executed during the startup of an operating system (OS), various settings information, and the like.

The storage 23 stores programs for information processing by the processor 21, the OS, and the like. A database storing various data to be used in the execution of programs may also be constructed in the storage 23.

The touch panel 24 forms an input-output interface. The touch panel 24 includes a touch input receiver 25a and a display 25b. The touch input receiver 25a receives touch operations performed by the user on the touch panel 24. The display 25b is achieved with a liquid crystal display or the like.

The communication interface 27 connects the user terminal 200 to the networks 400, 500, and 700, and communicates with each external device. The communication interface 27 may include short-range communication interfaces such as Bluetooth(R) and Bluetooth Low Energy (BLE), and can communicate with the aerosol-generating device 100 and the wearable terminal 600, for example.

The image capturer 28 is configured as an inward-facing camera and/or an outward-facing camera in the user terminal 200. In other words, the image capturer 28 has a camera function and stores images and/or videos captured by the user in the storage 23.

The bus 29 interconnects elements and conveys address signals, data signals, and control signals, for example. It is understood by a person skilled in the art that the user terminal 200 may also include a variety of elements other than the above elements. For example, the user terminal 200 may include location information acquisition means for acquiring location information about the user terminal 200 and activity level acquisition means for acquiring the user's activity level (such as a number of steps or a distance traveled, for example).

<1-3. Server>

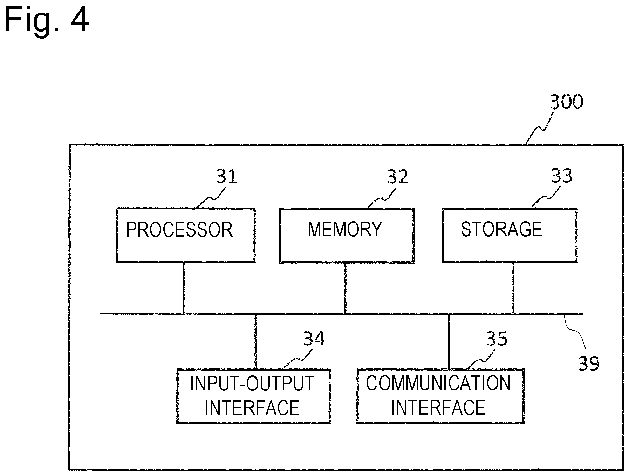

Fig. 4 is a schematic diagram of a hardware configuration of the server 300. The server 300 may be achieved using a general-purpose computer such as a workstation or a personal computer, for example. Alternatively, the server 300 may also be achieved logically by cloud computing.

The server 300 is provided with a processor 31, a memory 32, storage 33, an input-output interface 34, and a communication interface 35, the above elements being electrically connected to each other via a bus 39.

The processor 31, memory 32, storage 33, communication interface 35, and bus 39 are similar to the processor 21, memory 22, storage 23, communication interface 27, and bus 29 in the user terminal 200 illustrated in Fig. 3. In the server 300, the input-output interface 34 receives input from information input equipment such as a mouse and keyboard, and also provides output to output equipment such as a display.

## <1-4. Wearable terminal>

The wearable terminal 600 is an information processing device which is worn on the body of the user and which can continually acquire and store various activity levels including biometric information, and also provide the data to the user terminal 200 through communication. In one example, the wearable terminal 600 may be a smartwatch.

In one example, the wearable terminal 600 is provided with a processor 61, a memory 62, storage 63, a touch panel 64, a detector 66, a communication interface 67, and an image capturer 68, the above elements being electrically connected to each other via a bus 69. The touch panel 64 is provided with a touch input receiver 65a and a display 65b.

The processor 61 is a computational processing device that controls information processing, and is achieved by an electronic circuit such as a microprocessor in particular. Also, the image capturer 68 is achieved by a small camera or the like.

The memory 62, storage 63, touch panel 64 (touch input receiver 65a and display 65b), communication interface 67, and bus 69 are similar to the memory 22, storage 23, touch panel 24 (touch input receiver 25a and display 25b), communication interface 27, and bus 29 in the user terminal 200 illustrated in Fig. 3.

The detector 66 is achieved by internal sensors and includes, but is not limited to, a speed sensor, an acceleration sensor, a vibration sensor, a gyro sensor, a geomagnetic sensor, a proximity sensor, an illuminance sensor, a pressure sensor, a GPS, and the like. In addition, the detector 66 can function as an activity meter that measures the user's number of steps or distance traveled, for example. The detector 66 may also include a sensor such as a weather sensor.

Furthermore, the detector 66 includes a biometric sensor capable of acquiring biometric information about the user, such as an optical heart rate sensor, an electrocardiogram (ECG) sensor, or a myoelectric sensor. Through the use of such a biometric sensor, various types of health information about the user can be acquired. For example, a bioelectric signal can be acquired to measure heart rate, and in addition, the user's electrocardiogram, respiration, sleep state, and the like can be monitored. Otherwise, body temperature, blood pressure, blood sugar level, and the like may be measured through the use of any of various biometric sensors. In particular, according to one embodiment, the processor 61 can quantify the user's stress as a stress value by using data about the user's heart rate acquired by the detector 66.

Note that it is understood by a person skilled in the art that the health information about the user to be acquired by the detector 66 of the wearable terminal 600 is not limited to the above. Moreover, the biometric sensor described herein may be provided on the user terminal 200 side instead of, or in addition to, the wearable terminal 600.

## <2. First embodiments

### <2-1. Information processing system>

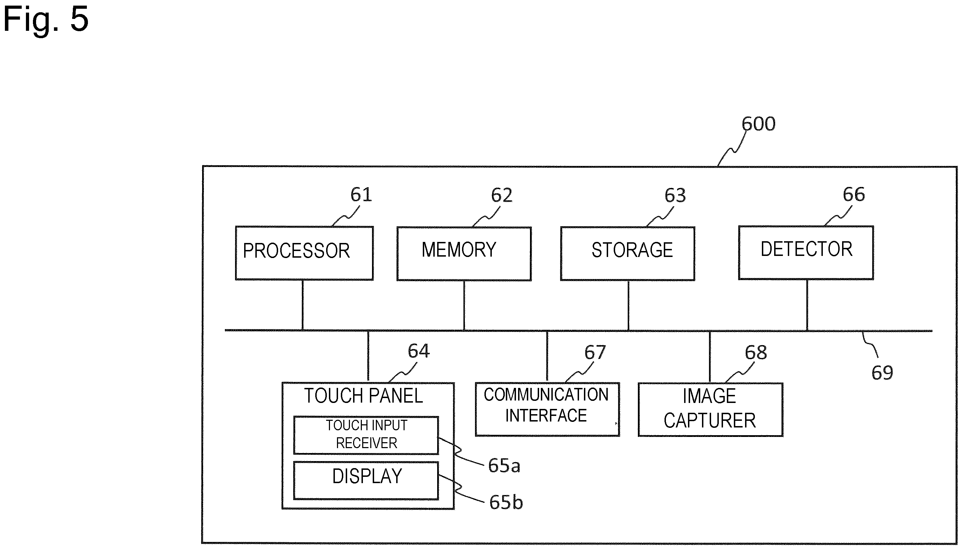

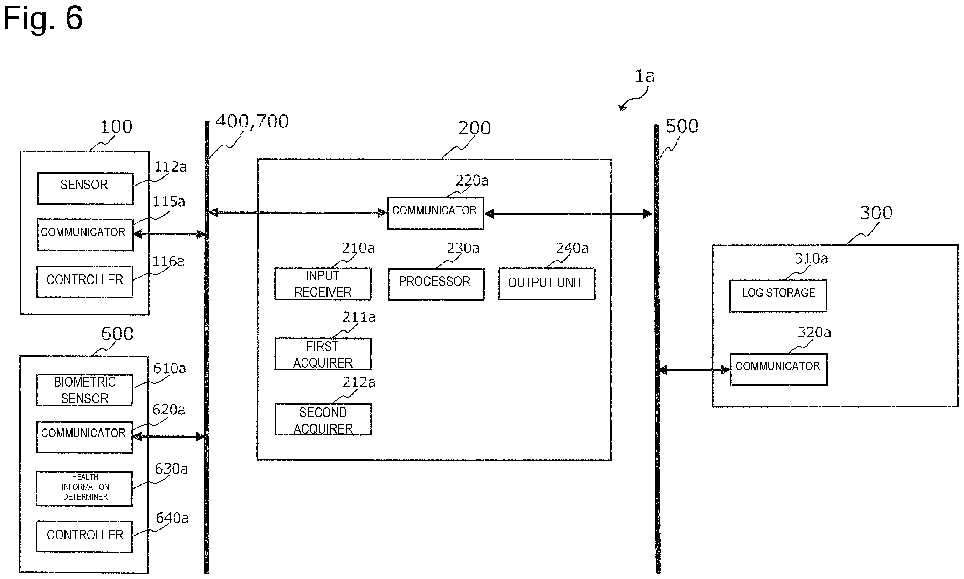

Fig. 6 is a schematic diagram of a functional configuration implemented in an information processing system 1a according to a first embodiment. Specifically, the information processing system 1a is provided with the aerosol-generating device 100 illustrated in Fig. 2, the user terminal 200 illustrated in Fig. 3, the server 300 illustrated in Fig. 4, and the wearable terminal 600 illustrated in Fig. 5. In Fig. 6, a major functional configuration implemented in each of the elements is illustrated as blocks. It should be understood by a person skilled in the art that the functional configuration is not limited thereto.

As illustrated in Fig. 2, the aerosol-generating device 100 is provided with a sensor 112a, a communicator 115a, and a controller 116a. The controller 116a causes the sensor 112a to acquire the puff count and puff operation time of the aerosol-generating device 100 and/or the consumption quantity (here, the used number) of the flavor-imparting cartridge 13, for example. The controller 116a also causes the communicator 115a to transmit the information to the user terminal 200.

The wearable terminal 600 is provided with a biometric sensor 610a, a communicator 620a, a health information determiner 630a, and a controller 640a.

The biometric sensor 610a is configured by the detector 66. Additionally, the controller 640a causes the biometric sensor 610a to acquire biometric information about the suer wearing the wearable terminal 600. In particular, the biometric sensor 610a may be configured to continually monitor and acquire the user's heart rate.

The controller 640a may also cause the health information determiner 630a to calculate a stress value on the basis of the user's heart rate acquired by the biometric sensor 610a. For example, the stress value may be determined by quantifying the heart rate as a percentage of a maximum heart rate. More specifically, the stress value may be calculated according to the following expression.

$$\text{Stress value} = \text{heart rate} \,/\, \text{maximum heart rate} \times 100$$

Here, the maximum heart rate can be estimated by using the following estimation formula.

$$\text{Maximum heart rate} = 220 - \text{user's age}$$

Note that the value of the user's age may be preregistered in the memory 62 when the user initially sets up the wearable terminal 600.

Additionally, the controller 640a causes the communicator 620a to transmit health information including the user's stress value to the user terminal 200.

The user terminal 200 forms an information processing terminal according to the first embodiment. The user terminal 200 is provided with an input receiver 210a, a first acquirer 211a, a second acquirer 212a, a communicator 220a, a processor 230a, and an output unit 240a.

The input receiver 210a receives the input of various information from the user. For example, the input of a stress sign (for example, an upper limit value on the stress value treated as a sign) is received from the user. The input receiver 210a may also receive input for editing a previously inputted stress sign.

The first acquirer 211a acquires usage information about the use of the aerosol-generating device 100 by the user from the aerosol-generating device 100 through communication. In particular, the usage information may include a usage period of the aerosol-generating device 100. More specifically, the usage information may include a usage start time and/or a usage end time of a puff operation of the aerosol-generating device 100. The usage information may also include a puff operation time from the beginning to the end of a series of puff operations by the user. Furthermore, the usage information may include a puff count by the user on the aerosol-generating device 100. The above usage information is data detected by the sensor 112a of the aerosol-generating device 100.

The second acquirer 212a acquires health information about the user from the wearable terminal 600. The health information about the user may include the user's heart rate and/or stress value described above which is calculated on the basis of the user's heart rate. The above information is data detected by the biometric sensor 610a or determined by the health information determiner 630a of the wearable terminal 600.

The processor 230a associates the health information about the user with the usage information about the aerosol-generating device 100. More specifically, the processor 230a may associate health information specified at a time later than the usage period of the aerosol-generating device 100 with a record of the use of the aerosol-generating device 100. The processor 230a may also associate the user's stress value with the usage information about the aerosol-generating device 100. Furthermore, the processor 230a may also associate the health information with the puff count for a series of puff operations by the user. In addition, the processor 230a may determine the change between the stress value when the aerosol-generating device 100 is used and the stress value after the aerosol-generating device 100 is used with respect to the record of the use of the aerosol-generating device 100, and associate the determined change with the usage information about the aerosol-generating device 100.

In response to a user request, the output unit 240a generates and outputs a daily activity screen regarding the usage information about the use of the aerosol-generating device 100 by the user and the user's state of health. According to one embodiment, the daily activity screen includes a record of the use of the aerosol-generating device 100 based on the usage information about the aerosol-generating device 100 and a display of a graph illustrating how the user state based on the health information about the user varies over time. In particular, variations in the user state may include variations in the stress value.

The output unit 240a may also output the change in the stress value with respect to the record of the use of the aerosol-generating device 100 by the user. Furthermore, the output unit 240a may output a daily total puff operation time and a consumption quantity of the aerosol-generating device 100 (for example, the used number of the flavor-imparting cartridge 13) calculated on the basis of the daily total puff count.

The server 300 is provided with log storage 310a and a communicator 320a. The log storage 310a stores various log information processed and acquired by the user terminal 200. The log information is used to present to the user a change in the state of health associated with the use of the aerosol-generating device 100, including the various information described above. Otherwise, the log information may also include location information and the like acquired by the user terminal 200.

<2-2. Process flow example>

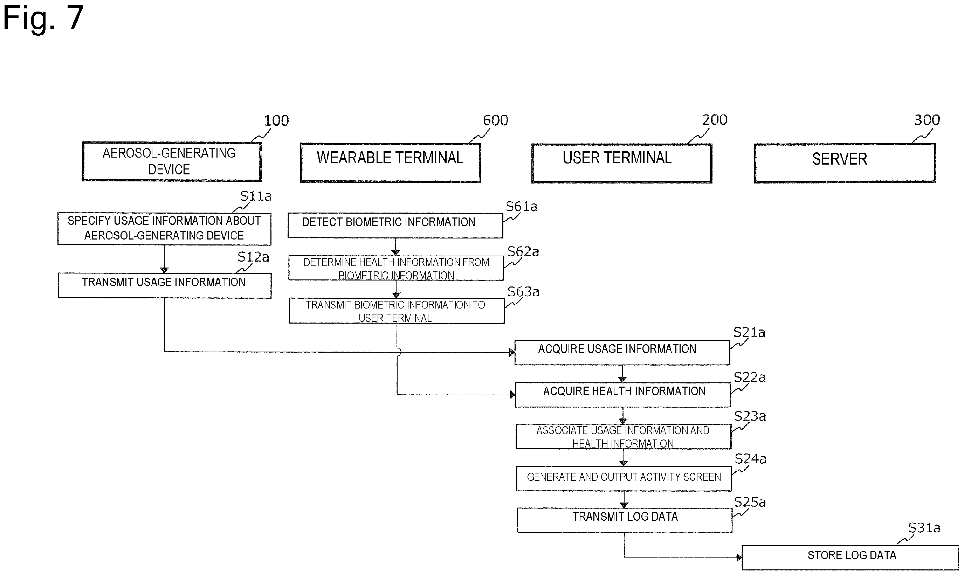

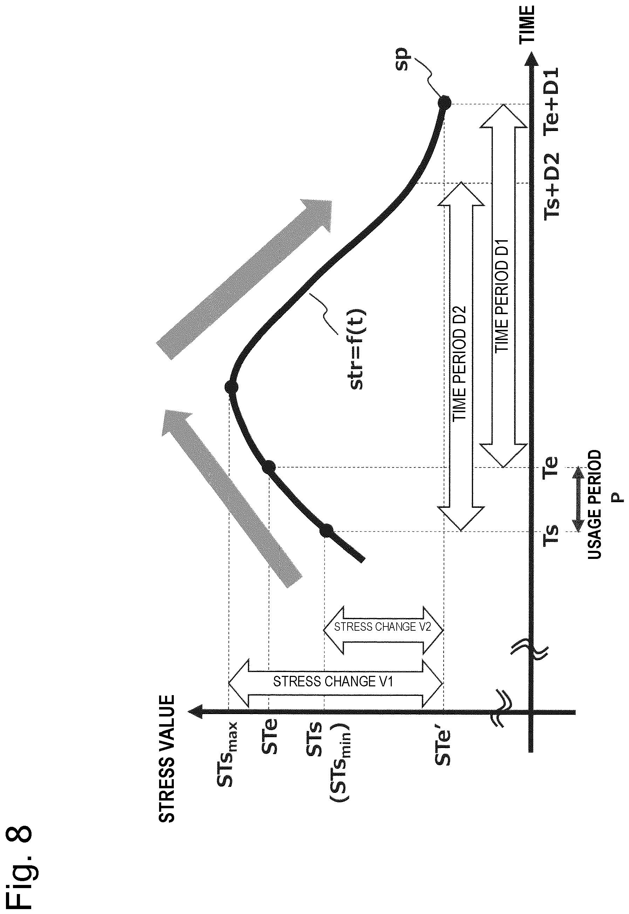

Figs. 7 and 8 will be referenced to describe general operations by the information processing system 1a according to the first embodiment. Fig. 7 is a process flowchart of the information processing system according to the first embodiment. Fig. 8 is an enlarged view of one example of a stress curve for specifying a change in the state of health associated with the use of the aerosol-generating device 100.

The following illustrates an example in which an information processing device, namely the user terminal 200, according to the present embodiment executes processing steps in cooperation with the aerosol-generating device 100, the wearable terminal 600, and the server 300 through communication. It is understood by a person skilled in the art that each of the processing steps illustrated herein is merely a non-limiting example, and any other processing steps may also be included. Moreover, the sequence of the processing steps illustrated herein is merely a non-limiting example, and in some cases, the processing steps may also be executed in a different sequence.

Every time the user uses the aerosol-generating device 100, that is, every time the user performs a series of puff operations, in step S11a, the sensor 112a of the aerosol-generating device 100 acquires usage information about the aerosol-generating device 100. The acquired information is temporarily stored in the storage 114 of the aerosol-generating device 100.

Next, in step S12a, the communicator 115a transmits the usage information about the use of the aerosol-generating device 100 by the user to the user terminal 200. Such transmission may be executed every time communication is established and interaction occurs between the aerosol-generating device 100 and the user terminal 200, for example. At this point, it is sufficient to transmit only the difference from the most recently transmitted usage information.

In response, in the user terminal 200 in step S21a, the communicator 220a receives the usage information transmitted from the aerosol-generating device 100, and the first acquirer 211a acquires the usage information. The acquired usage information is stored in the memory 22 or the like of the user terminal. For example, the usage information includes one or more of the usage period, the puff count, or the puff operation time of the aerosol-generating device 100.

On the other hand, in step S61a, the biometric sensor 610a of the wearable terminal 600 continually monitors and detects biometric information about the user wearing the wearable terminal 600. Such biometric information includes the user's heart rate. The detected biometric information is temporarily stored in the memory 62 or the like of the wearable terminal 600.

Next, in step S62a, the health information determiner 630a determines health information about the user on the basis of the biometric information. For example, the user's stress value is calculated on the basis of the user's heart rate acquired by the biometric sensor 610a. The health information about the user is also temporarily stored in the memory 62 or the like of the wearable terminal 600.

Next, in step S63a, the communicator 620a transmits the health information about the user determined in step S62a to the user terminal 200. Note that the biometric information acquired in step S61a may also be transmitted directly as the health information about the user. In this case, health information about the user may also be determined on the basis of the acquired biometric information on the user terminal 200 side instead of the health information determiner 630a of the wearable terminal 600.

The wearable terminal 600 and the user terminal 200 may communicate continuously while the user is wearing the wearable terminal 600 and the biometric information is being monitored. In addition to or instead of the above, communication between the two may be established periodically or may be established in response to a connection request from the user on either device. In other words, such transmission may be executed at any timing while communication is established and interaction is occurring between the wearable terminal 600 and the user terminal 200, for example. At this point, it is sufficient to transmit only the difference from the most recently transmitted health information.

In response, in the user terminal 200 in step S22a, the communicator 220a receives the health information transmitted from the wearable terminal 600, and the second acquirer 212a acquires the health information. The acquired health information is stored in the memory 22 or the like of the user terminal. It should be understood by a person skilled in the art that the sequential relationship of steps S21a and S22a is not limited to the above.

In step S23a, the processor 230a associates the health information acquired in step S22a with the usage information acquired in step S21a. More specifically, the use of the aerosol-generating device 100 by the user is associated with the user's state of health according to a history of use. The associated information is stored in the memory 62 and/or the storage 63.

One example of the relationship between a record of the use of the aerosol-generating device 100 based on the usage information acquired in step S21a and the health information acquired in step S22a is illustrated by the stress curve in Fig. 8. It is understood by a person skilled in the art that variations in the user state associated with the use of the aerosol-generating device 100 are specified by referring to the stress curve. Here, the stress curve str = f(t) is a function for which the horizontal axis is defined by the time t and the vertical axis is defined by a stress value str.

On the horizontal axis, a usage start time Ts, a usage end time Te, and a usage period P between the two times are specified. The usage start time Ts, the usage end time Te, and the usage period P are included in the usage information.

For example, the usage start time Ts may be taken to be the time at which the powering on of the aerosol-generating device 100 is detected by the sensor 112a. Similarly, the usage end time Te may be taken to be the time at which the powering off of the aerosol-generating device 100 is detected. Alternatively, the usage end time Te may be taken to be the time when an operation of some kind is not detected for a predetermined period (for example, two minutes) after the detection of the last operation performed by the user on the aerosol-generating device 100. The operations in this case may include puff operations.

As illustrated in Fig. 8, in some cases, the stress value briefly continues to increase immediately after the user uses the aerosol-generating device 100, and after a fixed time passes, the user's stress value decreases below the stress value before the use. The decrease in the user's stress value to below the stress value before the use is thought to be caused by the use of the aerosol-generating device 100 by the user. That is, the stress value that decreases as the fixed time passes may be associated with a record of the use of the aerosol-generating device 100 by the user.

More specifically, compared to the usage period P during which the user was actually using the aerosol-generating device 100, the stress value str briefly continues to increase even after the usage end time Te. Thereafter, the stress value reaches a maximum value $STs_{max}$ (which is also a local maximum in this case), after which the stress value decreases. Additionally, the stress value STe' after the passage of a time period D1 from the usage end time Te is a lower value than the stress values in the usage period P (for example, the stress value Ste at the usage end time Te). Note that according to this experiment by the inventor, such a time period D1 is approximately 30 minutes, for example.

In the present embodiment, in step S23a described above, the stress value specified at a time after the usage period P (that is, the time Te + D1) is associated with the record of the use of the aerosol-generating device 100 specified by the usage period P. With this arrangement, the reduction in the user's stress in accordance with the user using the aerosol-generating device 100 can be specified using a specific stress value.

Also, in step S23a described above, the change between the stress value when the aerosol-generating device 100 is used and the stress value after the aerosol-generating device 100 is used may be calculated with respect to the record of the use of the aerosol-generating device 100. With this arrangement, the change in the stress value before and after the use of the aerosol-generating device 100 can be associated with the record of use. That is, the reduction in the user's stress before and after the use of the aerosol-generating device 100 can be specified more effectively.

The stress value when the aerosol-generating device 100 is used may be taken to be the stress value STs at the usage start time Ts or the stress value STe at the usage end time Te. Alternatively, the stress value when the aerosol-generating device 100 is used may be determined on the basis of a stress value up until the time (that is, the time Ts + D2) after the passage of predetermined time period D2 from the usage start time Ts.

Specifically, the stress value when the aerosol-generating device 100 is used may be taken to be the "maximum value" (in the example in Fig. 8, $STs_{max}$) of the stress value up until the time (that is, the time Ts + D2) after the passage of predetermined time period D2 from the usage start time Ts. In another example, the stress value when the aerosol-generating device 100 is used may be taken to be the "minimum value" (in the example in Fig. 8, $STs_{min}$) of the stress value up until the time (that is, the time Ts + D2) after the passage of predetermined time period D2 from the usage start time Ts.

In the case where the "maximum value" described above is adopted as the stress value when the aerosol-generating device 100 is used, the change between the stress value when the aerosol-generating device 100 is used and the stress value after the aerosol-generating device 100 is used increases (stress change V1). In other words, the reduction in the stress value can be presented to the user more effectively. On the other hand, in the case where the "minimum value" described above is adopted as the stress value when the aerosol-generating device 100 is used, a change based on the most realistic and accurate stress value is calculated (stress change V2). In other words, more accurate health information can be presented to the user.

Note that, as described above, the stress value after the aerosol-generating device 100 is used should be the stress value (STe') at a time (that is, the time Te + D1) after the passage of a predetermined period from the usage end time.

In the example in Fig. 8, the usage information includes the usage period P, the usage start time Ts, and the usage end time Te. In addition to the above, the usage information may also include the number of puffs that the user takes using the aerosol-generating device 100, together with the puff operation time. In this case, in step S23a described above, the processor 230a may associate health information such as the stress value with the puff count for a series of puff operations by the user (that is, the number of puffs that the user takes during a single inhalation event). Similarly, the usage information may also include the puff operation time from the beginning to the end of a series of puff operations by the user.

Returning to Fig. 7, in step S24a subsequent to step S23a, the output unit 240a generates and outputs a daily user activity screen in response to a request from the user. The activity screen contains the various data processed in step S23a organized as daily activity data, and is presented to the user in response to a request from the user. For example, the activity screen may include a display of a graph in which a record of the use of the aerosol-generating device 100 based on the usage information and variations in the user state based on the health information are superimposed and illustrated over time.

Also, in step S24a, the activity screen outputted by the output unit 240a may further include a display of the change in the stress value described above with respect to the record of the use of the aerosol-generating device 100. Note that details about such an activity screen will be described later.

Finally, in step S25a, the communicator 220a transmits various data obtained by executing steps S21a to S24a to the server 300 as log data. The log data may include various data such as location information about the user terminal 200 and the like.

In response, in step S31a, the communicator 320a of the server 300 receives the log data and the log storage 310a stores the received log data in the memory 32 and/or the storage 33.

According to the present process flow, a change in the state of health associated with the use of the aerosol-generating device 100 can be presented to the user effectively. In particular, the present process flow is advantageous in that a reduction in the stress felt by the user before and after the user uses the aerosol-generating device 100 can be presented to the user in a visual way.

<2-3. Activity screen implementation examples>

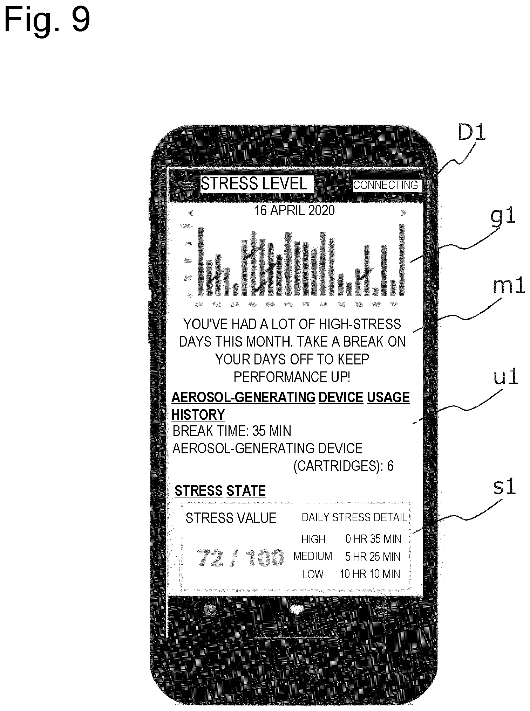

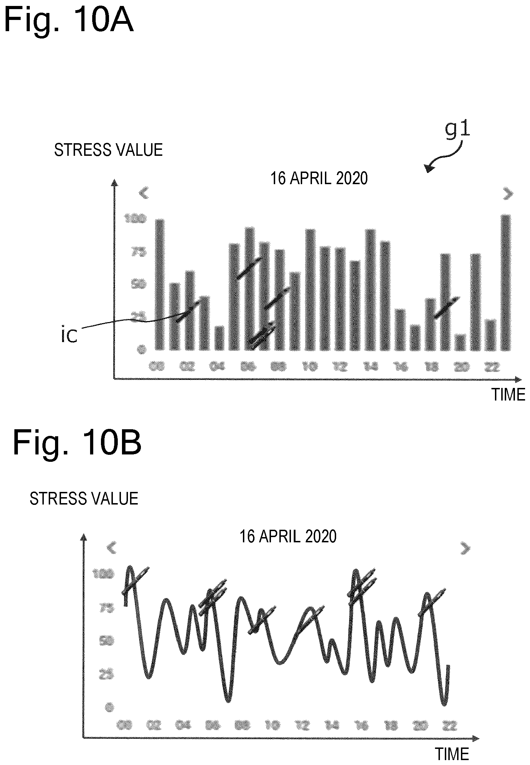

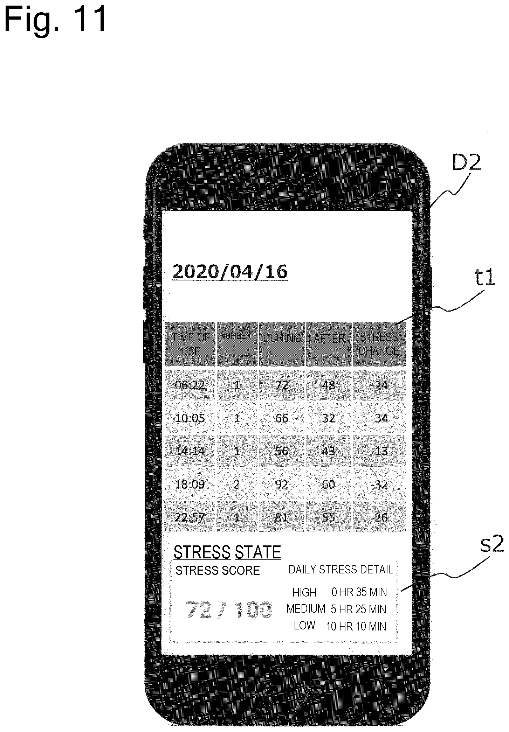

Hereinafter, Figs. 9 to 11 will be referenced to describe implementation examples of screen images generated by the output unit 240a of the user terminal 200 as a result of the processing in the process flow illustrated in Fig. 6. Figs. 9 and 11 are each an example of an activity screen image displayed on the user terminal 200. Also, Figs. 10A to 10C are several examples of graphs displayed on the activity screen. The user can select and view a desired activity screen from among Figs. 9 to 11 on the application 250. Note that it is also possible to implement only one screen in either Fig. 9 or Fig. 11.

Fig. 9 is an image of one example of an activity screen D1 generated by the output unit 240a in step S24a. The activity screen D1 includes a display of each of a graph g1, a message m1, aerosol-generating device 100 usage history information u1, and the user's stress state s1. The activity screen is generated according to daily aggregate data.

Fig. 10A is an enlarged image of a portion of the graph g1 illustrated in Fig. 9. In the graph g1, data is aggregated for a single day (in this example, 16 April 2020), with the horizontal axis representing time and the vertical axis representing the stress value. Additionally, a bar graph like the graph g1 illustrates variations in the average of the stress value for every hour of the day, and a record of the use of the aerosol-generating device 100 (herein, a tobacco icon ic) is superimposed and displayed together with the bar graph. For example, at 1:00 AM on 16 April 2020, the user's stress value indicates 50, and a record of the user using one flavor-imparting cartridge 13 of the aerosol-generating device 100 is presented in association with the stress value.

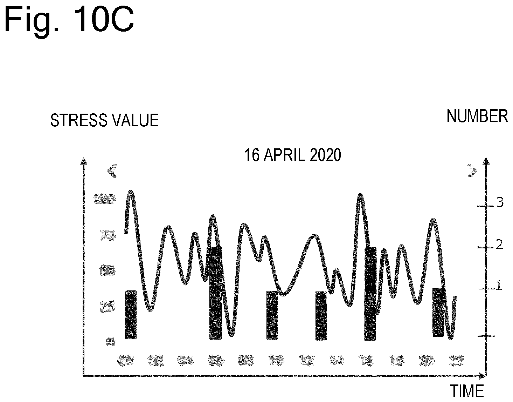

Fig. 10A illustrates a bar graph of the user's stress value by the hour, whereas Fig. 10B illustrates a continuous line graph of the user's stress value. In Fig. 10B, the stress value is plotted every 10 minutes, for example, and a curve is generated by interpolating between the plotted points. Additionally, a record of the use of the aerosol-generating device 100 is superimposed and displayed together with the line graph. Also, Fig. 10C is further provided with the consumption quantity of the aerosol-generating device 100 (herein, the used number of the flavor-imparting cartridge 13) on the vertical axis (right side) in addition to the line graph in Fig. 10B. That is, a bar graph indicating the used quantity of the aerosol-generating device 100 is superimposed and displayed together with the line graph of the stress value.

Returning to Fig. 9, with regard to the message m1 displayed on the activity screen D1, one message selected from among a plurality of prepared messages on the basis of the characteristics of the pattern of the stress value indicated by the graph g1 is displayed. The characteristics of the pattern of the stress value may be analyzed by the controller 640a of the wearable terminal 600 and supplied to the second acquirer 212a of the user terminal 200 as a part of the health information. Alternatively, the application 250 of the user terminal 200 may perform such analysis processing.

The aerosol-generating device 100 usage history information u1 displayed on the activity screen D1 is based on the usage information acquired by the second acquirer 212a. As described above, the usage information may include a puff operation time from the beginning to the end of a series of puff operations by the user. The break time of the usage history information u1 is calculated as the total time for the day when the puff operation times are aggregated for each day.

Similarly, the usage information may include the puff count for a series of puff operations by the user (that is, the number of puffs that the user takes during a single smoking event). Additionally, the used number for the aerosol-generating device (cartridges) in the usage history information u1 is the value obtained by aggregating and converting the puff counts for each day into a consumption quantity (that is, the used number) of flavor-imparting cartridges corresponding to the total puff count for the day. In this way, at least of a daily total puff count or a (cartridge) consumption quantity of the aerosol-generating device 100 calculated on the basis of the daily total puff count may be outputted. Note that the consumption quantity may be converted by considering 15 puffs to be equivalent to the consumption of one flavor-imparting cartridge 13, for example, but is not limited thereto.

The user's stress state s1 displayed on the activity screen D1 includes a display of the stress value and daily stress details. The stress value displayed on the activity screen D1 is the average score of the stress value for the day. Also, in the daily stress details, a time is displayed for each of "high", "medium", and "low" levels. In one non-limiting example, the times during which the stress value for the day was from 76 to 100, from 50 to 75, and from 0 to 49 are aggregated into the "high" level, the "medium" level, and the "low" level, respectively, and the total times are indicated for each of the levels.

Fig. 11 is an image of another example of an activity screen D2 generated in step S24a. In particular, on the activity screen D2, various data t1 is displayed in a table format instead of the graph g1 on the activity screen D1 in Fig. 9. Here, the "time of use" item is the usage start time (or the usage end time) of the aerosol-generating device 100 included in the usage information. Additionally, the data t1 is contained in a table chronologically according to the time of use.

The "number" item is the consumption quantity (that is, the used number) of the flavor-imparting cartridge 13 corresponding to the "time of use". The "during", "after", and "stress change" items are the stress value when the aerosol-generating device 100 is used, the stress value after the aerosol-generating device 100 is used, and the change (difference) between the two, which are respectively associated with the record of the use of the aerosol-generating device 100, as described with reference to Fig. 8.

Note that the display of the user's stress state s2 on the activity screen D2 is similar to the display of the user's stress state s1 on the activity screen D1.

In this way, on the activity screen D2, the output unit 240a outputs the change in the stress value with respect to the record of the use of the aerosol-generating device 100. With this arrangement, the user can check visualized variations in stress before and after the use of the aerosol-generating device 100 with objective, numerical values.

<3. Information processing system according to second embodiments

<3-1. Information processing system>

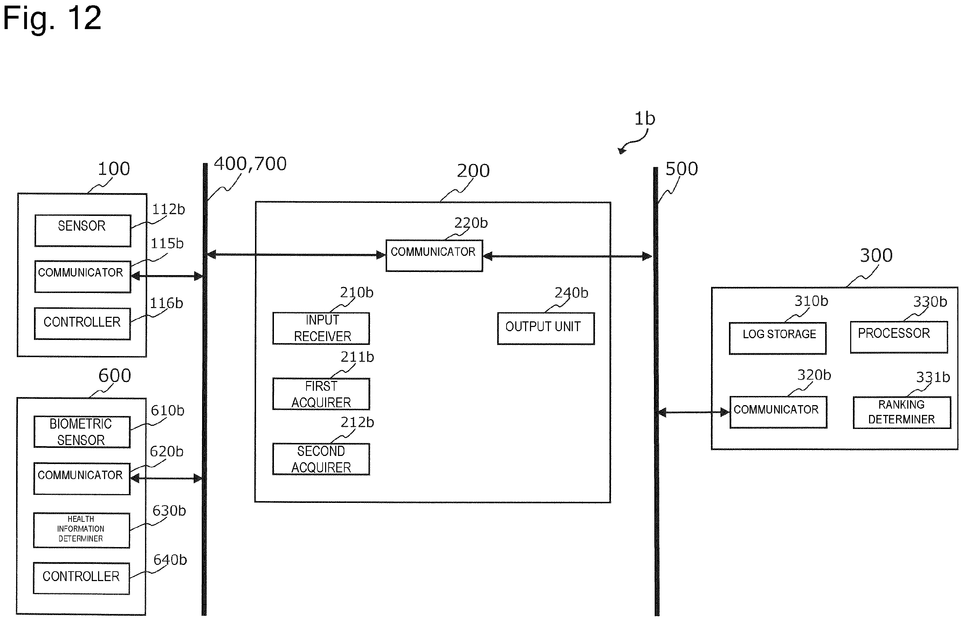

Fig. 12 is a schematic diagram of a functional configuration implemented in an information processing system 1b according to a second embodiment. Specifically, the information processing system 1b is provided with the aerosol-generating device 100, the user terminal 200, the server 300, and the wearable terminal 600, similarly to the information processing system 1a according to the first embodiment.

A sensor 112b, a communicator 115b, and a controller 116b of the aerosol-generating device 100 are similar to the sensor 112a, the communicator 115a, and the controller 116a of the aerosol-generating device 100 according to the first embodiment.

A biometric sensor 610b, a communicator 620b, a health information determiner 630b, and a controller 640b of the wearable terminal 600 are similar to the biometric sensor 610a, the communicator 620a, the health information determiner 630a, and the controller 640a of the wearable terminal 600 according to the first embodiment.

An input receiver 210b, a first acquirer 211b, a second acquirer 212b, a communicator 220b, and an output unit 240b of the user terminal 200 are similar to the input receiver 210a, the first acquirer 211a, the second acquirer 212a, the communicator 220a, and the output unit 240a of the user terminal 200 according to the first embodiment.

The server 300 includes log storage 310b, a communicator 320b, a processor 330b, and a ranking determiner 331b. Of the above, the log storage 310b and the communicator 320b are similar to the log storage 310a and the communicator 320a of the server 300 according to the first embodiment.

Functions similar to the processor 230a provided to the user terminal 200 in the first embodiment are implemented in the processor 330b of the server 300. The ranking determiner 331b of the server 300 determines a ranking (described later) of all registered users on the basis of a prescribed score.

<3-2. Process flow example>

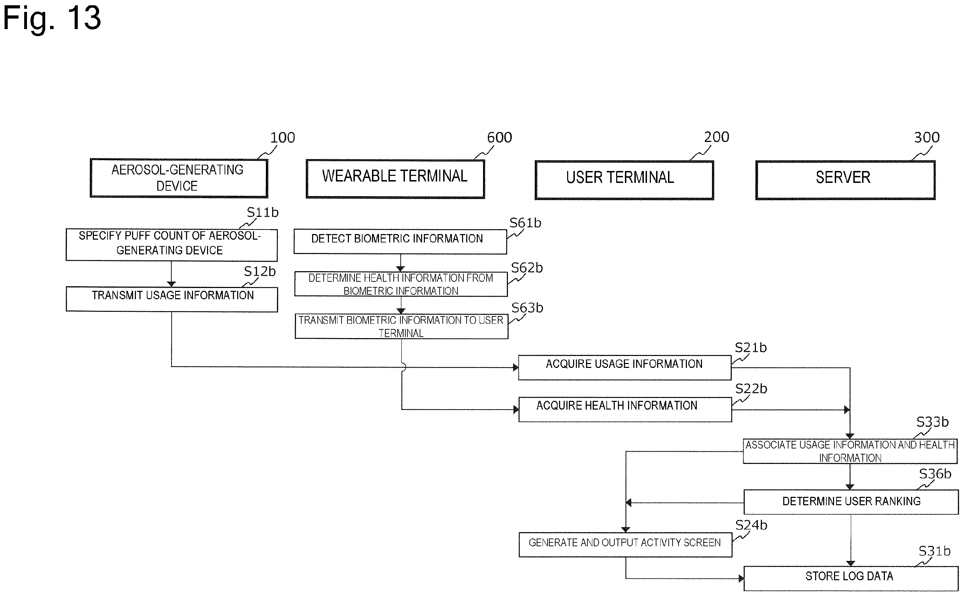

Fig. 13 is a process flowchart of the information processing system 1b according to the second embodiment. The following illustrates an example in which the user terminal 200 executes processing steps in cooperation with the aerosol-generating device 100, the wearable terminal 600, and the server 300 through communication. It is understood by a person skilled in the art that each of the processing steps illustrated herein is merely a non-limiting example, and any other processing steps may also be included. Moreover, the sequence of the processing steps illustrated herein is merely a non-limiting example, and in some cases, the processing steps may also be executed in a different sequence.

Steps S11b and S12b executed by the aerosol-generating device 100 are similar to steps S11a and S12a executed by the aerosol-generating device 100 in the first embodiment.

Also, steps S61b, S62b, and S63b executed by the wearable terminal 600 are similar to steps S61a, S62a, and S63a executed by the wearable terminal 600 in the first embodiment.

Furthermore, steps S21b and S22b executed by the user terminal 200 are similar to steps S21a and S22a executed by the user terminal 200 in the first embodiment. Step S24b executed by the user terminal 200 is also broadly similar to step S24a executed by the user terminal 200 in the first embodiment. That is, in step S24b, the screens and display items therein similar to those in step S24a of the first embodiment are displayed. In addition to step S24a, in step S24b, the output unit 240b generates and outputs a ranking screen described later.

Step S31b (that is, the step for storing log data) executed by the server 300 is similar to step S31a executed by the server 300 in the first embodiment.

Step S33b executed by the server 300 is similar to the functions of step S23a executed by the user terminal 200 in the first embodiment. That is, in the present embodiment, the server 300 is configured to perform the process for associating usage information and health information that is executed by the user terminal 200 in the first embodiment. For this reason, in the present embodiment, the exchange of data is executed as appropriate between the communicator 220b of the user terminal 200 and the communicator 320b of the server 300.

Next, in step S36b executed by the server 300, the ranking determiner 331b determines a user ranking on the basis of the health information about the user. For example, an average of the stress value for one week calculated from the daily stress values for a certain user may be used to determine the ranking of the user among all users. Note that the determined user ranking may be displayed by the output unit 240b of the user terminal 200.

According to the present process flow, a change in the state of health associated with the use of the aerosol-generating device 100 can be presented to the user effectively. In particular, a reduction in the stress felt by the user before and after the user uses the aerosol-generating device 100 can be presented to the user in a visual way.

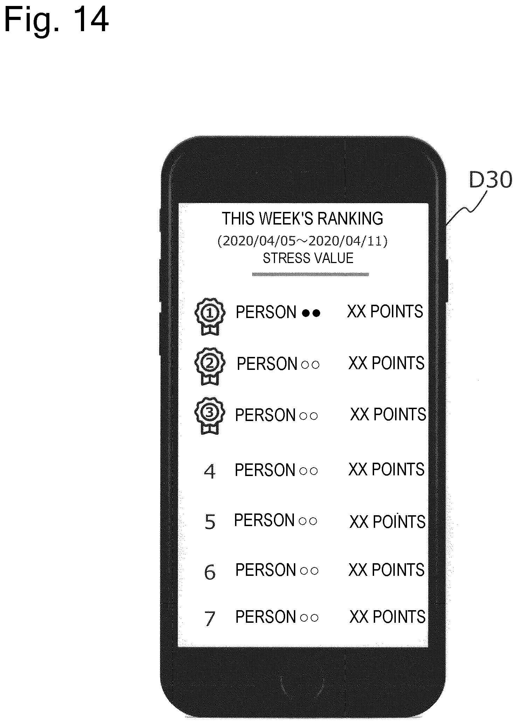

Fig. 14 is an example image of a ranking screen D3 displayed on the user terminal 200 in step S24b. In this case, the stress values of users for the week from 5 April 2020 to 11 April 2020 are ranked. Here, the stress values are ranked such that smaller stress values are ranked higher. The stress values may be obtained by using daily stress values to calculate an average for the week.

<4. Other embodiments>

(1) In the above description of the first and second embodiments, the heart rate is monitored and detected by the wearable terminal 600 as one example of biometric information about the user. Instead of the above, in another embodiment, the user terminal 200 may detect the user's heart rate. In the wearable terminal 600, a light-emitting diode (LED) light and a photosensitive photodiode can also be used to measure heart rate. On the basis of this mechanism, for example, the image capturer 28 of the user terminal 200 may be used to cause a flashlight (not illustrated) to blink in response to a finger touching the image capturer 28 configured as an outward-facing camera, and by measuring the intensity of the reflected light, the user's heart rate is measurable in detail in the user terminal 200.

In this case, the biometric sensor 610a or 610b of the wearable terminal 600 may also be provided to the user terminal 200. Also, in this case, the stress value determined from the user's heart rate is determined by the user terminal 200. That is, the health information determiner 630a or 630b of the wearable terminal 600 may also be provided to the user terminal 200.

(2) In the above description of the second embodiment, the server 300 mainly cooperates with the user terminal 200 to execute information processing including the process for further associating usage information and health information. In addition to the above, the server 300 may also be linked to an external web service and perform additional information processing such as associating usage information and health information with information acquired from the external web service. For example, weather information may be acquired from an external source, and variations in the weather may be associated with the user state based on the health information and displayed on the user terminal 200. With this arrangement, the relationship between the weather information and the user's state of health can be visualized on the user terminal 200.

(3) In the above description, an information processing device, an information processing method, and an information processing system according to several embodiments are described with reference to the drawings. It is understood that the present disclosure may also be carried out as a program that, when executed by a processor, causes the processor to operate as the information processing device and/or execute the information processing method, or may be carried out as a computer-readable storage medium storing the program.

The foregoing describes embodiments of the present disclosure along with modifications and applications thereof, but it should be understood that the above examples are merely for illustrative purposes and do not limit the scope of the present disclosure. It should be understood that changes, additions, improvements, and the like can be made to the embodiments without departing from the gist and scope of the present disclosure. The scope of the present disclosure should not be limited by any of the embodiments described above, but should be defined only by the claims and their equivalents.

REFERENCE SIGNS LIST

11 power supply unit
12 cartridge
13 flavor-imparting cartridge
100 aerosol-generating device
112, 112a, 112b sensor
115, 115a, 115b communicator
116, 116a, 116b controller
200 user terminal
300 server
210a, 210b input receiver
211a, 211b first acquirer
212a, 212b second acquirer
220a, 220b communicator
230a, 330b processor
240a, 240b output unit
250 application
310a, 310b log storage
320a, 320b communicator
331b ranking determiner
600 wearable terminal
610a, 610b biometric sensor
620a, 620b communicator
630a health information determiner
640a, 640b controller
400, 500, 700 network
D1, D2 activity screen
D3 ranking screen

**Claims**

1.   An information processing device comprising:

a first acquirer that acquires usage information about a use of an inhalation device by a user from the inhalation device;
a second acquirer that acquires health information about the user from a wearable terminal;
a processor that associates the health information with the usage information; and
an output unit that generates and outputs a daily activity screen in response to a request from the user,
the activity screen including a display of a graph in which a record of the use of the inhalation device based on the usage information and variations in a user state based on the health information are illustrated over time.

2. The information processing device according to claim 1, wherein

the usage information includes a usage period of the inhalation device, and
the processor is configured to associate the health information specified at a time later than the usage period with the record of the use of the inhalation device.

3. The information processing device according to claim 1 or 2, wherein

the processor is configured to associate with the usage information a stress value calculated on a basis of a heart rate of the user monitored by the wearable terminal, and
the variations in the user state are variations in the stress value.

4. The information processing device according to claim 3, wherein

the processor is further configured to determine a change between the stress value when the inhalation device is used and the stress value after the inhalation device is used with respect to the record of the use of the inhalation device, and
the output unit is further configured to output the change in the stress value with respect to the record of the use of the inhalation device.

5. The information processing device according to claim 4, wherein

the usage information includes a usage end time of the inhalation device, and
the stress value after the inhalation device is used is the stress value after the passage of a predetermined first time period from the usage end time.

6. The information processing device according to claim 5, wherein

the usage information further includes a usage start time of the inhalation device, and
the stress value when the inhalation device is used is the stress value at the usage start time or the usage end time.

7. The information processing device according to claim 5, wherein

the usage information further includes a usage start time of the inhalation device, and
the stress value when the inhalation device is used is a maximum value or a minimum value of the stress value from the usage start time until the passage of a predetermined second time period.

8. The information processing device according to any one of claims 1 to 7, wherein

the inhalation device is an aerosol-generating device,
the usage information includes a puff count for the aerosol-generating device, and
the processor is configured to associate the health information with the puff count for a series of puff operations by the user.

9. The information processing device according to claim 8, wherein

the usage information includes a puff operation time from a beginning to an end of the series of puff operations by the user, and
the output unit is further configured to output a daily total time of the puff operation time.

10. The information processing device according to claim 8 or 9, wherein
the output unit is further configured to output a consumption quantity of the aerosol-generating device calculated on a basis of a daily total of the puff count.

11. An information processing method comprising:

acquiring usage information about a use of an inhalation device by a user from the inhalation device and acquiring health information about the user from a wearable terminal;
associating the health information with the usage information; and
generating and outputting a daily activity screen in response to a request from the user,
the activity screen including a display of a graph in which a record of the use of the inhalation device based on the usage information and variations in a user state based on the health information are illustrated over time.

12. The information processing method according to claim 11, wherein

the usage information includes a usage period of the inhalation device, and

the associating step is configured to associate the health information specified at a time later than the usage period with the record of the use of the inhalation device.

13. The information processing method according to claim 11 or 12, wherein

the associating step is configured to associate with the usage information a stress value calculated on a basis of a heart rate of the user monitored by the wearable terminal, and
the variations in the user state are variations in the stress value.

14. The information processing method according to claim 13, wherein

the associating step further includes determining a change between the stress value when the inhalation device is used and the stress value after the inhalation device is used with respect to the record of the use of the inhalation device, and
the outputting step further includes outputting the change in the stress value with respect to the record of the use of the inhalation device.

15. The information processing method according to claim 14, wherein

the usage information includes a usage end time of the inhalation device, and
the stress value after the inhalation device is used is the stress value after the passage of a predetermined first time period from the usage end time.

16. The information processing method according to claim 15, wherein

the usage information further includes a usage start time of the inhalation device, and
the stress value when the inhalation device is used is the stress value at the usage start time or the usage end time.

17. The information processing method according to any one of claims 11 to 16, wherein

the inhalation device is an aerosol-generating device,
the usage information includes a puff count for the aerosol-generating device, and
the associating step is configured to associate the health information with the puff count for a series of puff operations by the user, and
the outputting step further includes outputting at least one of a daily total of the puff count or a consumption quantity of the aerosol-generating device calculated on a basis of the daily total of the puff count.

18. The information processing method according to claim 17, wherein

the usage information includes a puff operation time from a beginning to an end of the series of puff operations by the user, and
the outputting step further includes outputting a daily total time of the puff operation time.

19. An information processing system comprising a user terminal and a server,

the user terminal including:

a first acquirer that acquires usage information about a use of an inhalation device by a user from the inhalation device;
a second acquirer that acquires health information about the user from a wearable terminal;
an output unit that generates and outputs a daily activity screen in response to a request from the user, the server including a processor that associates the health information with the usage information,

the activity screen including a display of a graph in which a record of the use of the inhalation device based on the usage information and variations in a user state based on the health information are illustrated over time.

20. The information processing system according to claim 19, wherein

the server further includes a ranking determiner that determines a ranking of the user on a basis of the health information associated with the usage information, and
the activity screen to be displayed by the output unit of the user terminal is configured to display the ranking of the user.

# Fig. 1

1

100

400

200

250

APPLICATION

500

300

700

600

Fig. 2

# Fig. 3

# Fig. 4

# Fig. 5

600

| 61 | 62 | 63 | 66 |
|---|---|---|---|
| PROCESSOR | MEMORY | STORAGE | DETECTOR |

69

64

**TOUCH PANEL**

TOUCH INPUT
RECEIVER

65a

DISPLAY

65b

67

COMMUNICATION
INTERFACE

68

IMAGE
CAPTURER

# Fig. 6

EP 4 260 741 A1

# Fig. 7

EP 4 260 741 A1

# Fig. 8

STRESS VALUE

$STs_{max}$

STe

STs
($STs_{min}$)

STRESS CHANGE V1

STRESS CHANGE V2

str=f(t)

sp

STe'

TIME PERIOD D2

TIME PERIOD D1

TIME

Ts      Te

Ts+D2   Te+D1

USAGE PERIOD
P

# Fig. 9

# Fig. 10A

STRESS VALUE

16 APRIL 2020

g1

ic

TIME

# Fig. 10B

STRESS VALUE

16 APRIL 2020

TIME

# Fig. 10C

# Fig. 11

**2020/04/16**

| TIME OF USE | NUMBER | DURING | AFTER | STRESS CHANGE |
|---|---|---|---|---|
| 06:22 | 1 | 72 | 48 | -24 |
| 10:05 | 1 | 66 | 32 | -34 |
| 14:14 | 1 | 56 | 43 | -13 |
| 18:09 | 2 | 92 | 60 | -32 |
| 22:57 | 1 | 81 | 55 | -26 |

**STRESS STATE**

STRESS SCORE · · · · · · · · DAILY STRESS DETAIL

72 / 100

HIGH · · · 0 HR 35 MIN
MEDIUM · · 5 HR 25 MIN
LOW · · · 10 HR 10 MIN

Fig. 12

EP 4 260 741 A1

# Fig. 13

EP 4 260 741 A1

# Fig. 14

D30

THIS WEEK'S RANKING
(2020/04/05~2020/04/11)
STRESS VALUE

① PERSON ●●     XX POINTS

② PERSON ○○     XX POINTS

③ PERSON ○○     XX POINTS

4   PERSON ○○     XX POINTS

5   PERSON ○○     XX POINTS

6   PERSON ○○     XX POINTS

7   PERSON ○○     XX POINTS

## INTERNATIONAL SEARCH REPORT

| International application No. |
| --- |
| PCT/JP2020/046519 |

A. CLASSIFICATION OF SUBJECT MATTER
A24F 40/65(2020.01)i; A61B 5/00(2006.01)i; A61B 5/16(2006.01)i
FI: A61B5/00 G; A24F40/65; A61B5/16 110

According to International Patent Classification (IPC) or to both national classification and IPC

B. FIELDS SEARCHED

Minimum documentation searched (classification system followed by classification symbols)
A24F40/65; A61B5/00; A61B5/16

Documentation searched other than minimum documentation to the extent that such documents are included in the fields searched
Published examined utility model applications of Japan 1922–1996
Published unexamined utility model applications of Japan 1971–2021
Registered utility model specifications of Japan 1996–2021
Published registered utility model applications of Japan 1994–2021

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | JP 2020-68739 A (JAPAN TOBACCO INC.) 07 May 2020 (2020-05-07) paragraphs [0014]-[0051], fig. 7, 8 | 1-6, 8-19 |
| A | paragraphs [0014]-[0051], fig. 7, 8 | 7, 20 |
| Y | JP 2019-72486 A (SAMSUNG ELECTRONICS CO., LTD.) 16 May 2019 (2019-05-16) paragraphs [0066]-[0067], [0073], fig. 4, 6, 9 | 1-6, 8-19 |
| Y | JP 2006-507499 A (BRITISH AMERICAN TOBACCO (INVESTMENTS) LTD.) 02 March 2006 (2006-03-02) paragraph [0036] | 9, 18 |
| Y | JP 2019-509733 A (PHILIP MORRIS PRODUCTS S.A.) 11 April 2019 (2019-04-11) paragraph [0089] | 10, 17-18 |
| A | JP 2020-54606 A (SHARP CORP.) 09 April 2020 (2020-04-09) entire text, all drawings | 1-20 |

☐ Further documents are listed in the continuation of Box C.   ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 26 January 2021 (26.01.2021) | 09 February 2021 (09.02.2021) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

<div align="center">

**INTERNATIONAL SEARCH REPORT**
Information on patent family members

</div>

| International application No. |
| --- |
| PCT/JP2020/046519 |

| Patent Documents referred in the Report | Publication Date | Patent Family | Publication Date |
| --- | --- | --- | --- |
| JP 2020-68739 A | 07 May 2020 | KR 10-2020-0050868 A<br>CN 111134365 A | |
| JP 2019-72486 A | 16 May 2019 | US 2019/0115107 A1<br>paragraphs [0094]-[0095], [0104]-[0105], fig. 4, 6, 9<br>KR 10-2019-0043319 A<br>CN 111225603 A | |
| JP 2006-507499 A | 02 Mar. 2006 | US 2006/0099554 A1<br>paragraph [0044] | |
| JP 2019-509733 A | 11 Apr. 2019 | US 2017/0245552 A1<br>paragraph [0106] | |
| JP 2020-54606 A | 09 Apr. 2020 | (Family: none) | |

Form PCT/ISA/210 (patent family annex) (January 2015)

**REFERENCES CITED IN THE DESCRIPTION**

**Patent documents cited in the description**

- WO 2019175810 A
- WO 2016199065 A
- WO 2018098371 A